# EUROPEAN PATENT APPLICATION

(11) **EP 4 134 434 A1**
(43) Date of publication of application: **15.02.2023**
(21) Application number: 21785249.0
(22) Date of filing: 08.04.2021
(51) Int. Cl.: C12N 15/54, C12N 1/20, C12N 1/21, C12N 15/62, C12P 7/62

(54) **METHOD FOR MANUFACTURING COPOLYMER POLYHYDROXYALKANOIC ACID MIXTURE, AND TRANSFORMED MICROORGANISM**

(30) Priority: 10.04.2020 JP 2020071054
(71) Applicant: Kaneka Corporation, Osaka-shi, Osaka 530-8288 (JP)
(72) Inventor: ARIKAWA, Hisashi, Takasago-shi, Hyogo (JP); SATO, Shunsuke, Takasago-shi, Hyogo (JP); FURUTATE, Sho, Takasago-shi, Hyogo (JP)
(74) Representative: Vossius & Partner Patentanwälte Rechtsanwälte mbB
(86) International application number: PCT/JP2021/014958
(87) International publication number: WO 2021/206155

(57) **Abstract**

Provided is a method of producing a polyhydroxyalkanoate copolymer mixture. The method includes the step of culturing a microorganism that produces the polyhydroxyalkanoate copolymer mixture. The mixture contains: a fraction (I) containing a polyhydroxyalkanoate copolymer having a 3-hydroxybutyrate structural unit and a 3-hydroxyhexanoate structural unit, the fraction (I) having an average 3-hydroxyhexanoate unit ratio of 20 mol% or more; and a fraction (II) containing a polyhydroxyalkanoate having a 3-hydroxybutyrate structural unit, the fraction (II) having an average 3-hydroxyhexanoate unit ratio of 0 to 15 mol%. The polyhydroxyalkanoate copolymer mixture has an average 3-hydroxyhexanoate unit ratio of 22 mol% or less.

## Description

### Technical Field

The present invention relates to a method of producing a polyhydroxyalkanoate copolymer mixture and to a transformed microorganism.

### Background Art

Polyhydroxyalkanoates (also referred to as "PHAs" hereinafter) are organic polyester polymers produced by a wide variety of microorganisms. PHAs are biodegradable thermoplastic polymers and producible using renewable sources as raw materials. For these reasons, attempts have been made to industrially produce PHAs as environmentally friendly materials or biocompatible materials and use the produced PHAs in diverse industrial fields.

At present, many microorganisms have been known to accumulate PHAs as energy storage substances in the microbial bodies. Typical examples of the PHAs include poly-3-hydroxybutyrate (also referred to as "P(3HB)" hereinafter) which is a homopolymer of 3-hydroxybutyrate (also referred to as "3HB" hereinafter). P(3HB) is a thermoplastic polymer and biologically degradable in the natural environment. This is why P(3HB) has been attracting attention as an eco-friendly plastic. However, P(3HB) is hard and brittle because of its high crystallinity, and has limited practical application. It has been necessary to impart flexibility to P(3HB) in order to extend its application range.

Under these circumstances, a PHA copolymer composed of 3HB and 3-hydroxyvalerate (hereinafter referred to as "3HV") and a method of producing the PHA copolymer (hereinafter referred to as "P(3HB-co-3HV)") have been developed (see Patent Literature 1 and Patent Literature 2, for example). P(3HB-co-3HV) is more flexible than P(3HB) and thus had been considered usable in a wide variety of applications. In fact, however, increasing the 3HV mole fraction in P(3HB-co-3HV) leads to little change in physical properties and cannot increase the flexibility to a level required for processing into products such as, in particular, films, sheets, and soft packaging containers. Thus, P(3HB-co-3HV) has been used in only limited kinds of hard molded articles such as shampoo bottles and the handles of disposable razors.

In an attempt to increase the flexibility of PHAs, a polyhydroxyalkanoate copolymer composed of 3HB and 3-hydroxyhexanoate (hereinafter referred to as "3HH") and a method of producing the polyhydroxyalkanoate copolymer (also referred to as "P(3HB-co-3HH)" hereinafter) have been studied (see Patent Literature 3 and Patent Literature 4, for example). In these reports, P(3HB-co-3HH) is produced by fermentation which uses a wild strain of *Aeromonas caviae* isolated from soil and in which the carbon source is a fatty acid such as oleic acid or palmitic acid.

A study has also been conducted in which P(3HB-co-3HH) is highly produced by using *Cupriavidus necator* as a host and using a PHA synthase derived from *Aeromonas caviae.* In the P(3HB-co-3HH) production, a vegetable oil is used as a raw material, and the 3HH unit ratio in the P(3HB-co-3HH) is increased up to about 14 mol% by introducing (R)-specific enoyl-CoA hydratase gene into *Cupriavidus necator* having the *Aeromonas caviae*-derived PHA synthase or by enhancing the expression of the (R)-specific enoyl-CoA hydratase gene on the host chromosome (see Patent Literature 5, Patent Literature 6, and Non-Patent Literature 1).

There is also an example where the 3HH unit ratio in P(3HB-co-3HH) is increased to 20 mol% or more by inhibiting, in *Cupriavidus necator* having an *Aeromonas caviae*-derived PHA synthase, the expression of a gene encoding a β-ketothiolase enzyme having thiolysis activity for β-ketoacyl-CoA having six carbon atoms, i.e., β-ketohexanoyl-CoA (see Patent Literature 7).

A study about the physical properties of P(3HB-co-3HH) has also been conducted (see Non-Patent Literature 2). In this report, P(3HB-co-3HH) is produced with varying 3HH unit ratios by fermentation in which *Aeromonas caviae* is cultured using a fatty acid having 12 or more carbon atoms as a single carbon source. This report has revealed the following facts: the crystallinity of P(3HB-co-3HH) decreases as the 3HH unit ratio increases; thus, P(3HB-co-3HH), which is hard and brittle like P(3HB) when the 3HH unit ratio is low, becomes more flexible with increasing 3HH unit ratio; and P(3HB-co-3HH) exhibits higher flexibility than P(3HB-co-3HV) when the 3HH unit ratio is higher than a certain value. That is, the physical properties of P(3HB-co-3HH) can be varied widely from those suitable for use as a rigid polymer to those suitable for use as a soft polymer by changing the 3HH unit ratio. P(3HB-co-3HH) is therefore expected to be applicable to a wide variety of fields.

Increasing the 3HH unit ratio in P(3HB-co-3HH) provides a decrease in crystallinity and hence an improvement in flexibility. However, the increase in 3HH unit ratio tends to reduce the processability of P(3HB-co-3HH). For example, P(3HB-co-3HH) with an increased 3HH unit ratio of about 10 mol% is relatively soft, but the use of this P(3HB-co-3HH) in a process such as injection molding, film formation, blow molding, fiber spinning, extrusion foaming, or bead foaming results in low productivity due to slow rate of crystallization. With the goal of solving this problem, a study has been conducted in which the relatively soft P(3HB-co-3HH) and a PHA copolymer having a low 3HH unit ratio and a high melting point (i.e., high crystallinity) are produced together in the same cells to improve the melt processability and increase the processing rate (see Patent Literature 8).

However, in the PHA mixture described in Patent Literature 8, the low-melting component (PHA component considered to have the highest 3HH unit ratio) has a melting point higher than 100°C, and this leads to the inference that the PHA mixture does not contain any PHA component having a 3HH unit ratio as high as 20 mol% or more. The PHA mixture is insufficient in terms of mechanical properties such as tearing resistance and leaves room for improvement.

### Citation List

### Patent Literature

PTL 1: Japanese Laid-Open Patent Application Publication No. S57-150393
PTL 2: Japanese Laid-Open Patent Application Publication No. S59-220192
PTL 3: Japanese Laid-Open Patent Application Publication No. H5-93049
PTL 4: Japanese Laid-Open Patent Application Publication No. H7-265065
PTL 5: WO 2011/105379
PTL 6: WO 2015/115619
PTL 7: WO 2019/142845
PTL 8: WO 2017/056442

### Non-Patent Literature

NPL 1: H. Arikawa, K. Matsumoto, *Microb.Cell.Fact.,* 15, pp. 184 (2016)
NPL 2: Y. Doi, S. Kitamura, H. Abe, Macromolecules, 28, pp. 4822-4823 (1995)

### Summary of Invention

### Technical Problem

As described above, a molded article that is satisfactory in terms of both processability and mechanical properties has been difficult to obtain using P(3HB-co-3HH).

In view of the above circumstances, the present invention aims to provide a method of producing a polyhydroxyalkanoate copolymer having both excellent processability and excellent mechanical properties.

### Solution to Problem

As a result of intensive studies aimed at solving the above problems, the present inventors have found that when a microorganism is cultured and allowed to produce a polyhydroxyalkanoate copolymer mixture containing two kinds of polyhydroxyalkanoate fractions having particular compositions, the polyhydroxyalkanoate copolymer mixture obtained can have both excellent processability and excellent mechanical properties. Based on this finding, the inventors have completed the present invention.

Specifically, the present invention relates to a method of producing a polyhydroxyalkanoate copolymer mixture, the method including the step of culturing a microorganism that produces the polyhydroxyalkanoate copolymer mixture, wherein the polyhydroxyalkanoate copolymer mixture contains: a polyhydroxyalkanoate fraction (I) containing a polyhydroxyalkanoate copolymer having a 3-hydroxybutyrate structural unit and a 3-hydroxyhexanoate structural unit, the polyhydroxyalkanoate fraction (I) having an average 3-hydroxyhexanoate unit ratio of 20 mol% or more; and a polyhydroxyalkanoate fraction (II) containing a polyhydroxyalkanoate having a 3-hydroxybutyrate structural unit, the polyhydroxyalkanoate fraction (II) having an average 3-hydroxyhexanoate unit ratio of 0 to 15 mol%, and the polyhydroxyalkanoate copolymer mixture has an average 3-hydroxyhexanoate unit ratio of 22 mol% or less.

Preferably, a weight percentage of the polyhydroxyalkanoate fraction (I) in the polyhydroxyalkanoate copolymer mixture is from 10 to 90%.

Preferably, the average 3-hydroxyhexanoate unit ratio of the polyhydroxyalkanoate copolymer mixture is from 10 to 22 mol%.

Preferably, the microorganism has genes encoding two types of polyhydroxyalkanoate synthases differing in polymerization activity for 3-hydroxyhexanoyl-CoA.

Preferably, amino acid sequences of the two types of polyhydroxyalkanoate synthases differing in polymerization activity for 3-hydroxyhexanoyl-CoA have a sequence identity of 90% or less.

Preferably, the genes encoding the two types of polyhydroxyalkanoate synthases differing in polymerization activity for 3-hydroxyhexanoyl-CoA include: a gene (A) encoding a polyhydroxyalkanoate synthase having higher polymerization activity for 3-hydroxyhexanoyl-CoA than an *Aeromonas caviae*-derived wild-type polyhydroxyalkanoate synthase having an amino acid sequence of SEQ ID NO: 1; and a gene (B) encoding a polyhydroxyalkanoate synthase having lower polymerization activity for 3-hydroxyhexanoyl-CoA than the *Aeromonas caviae*-derived wild-type polyhydroxyalkanoate synthase.

Preferably, the gene (A) is a polyhydroxyalkanoate synthase gene derived from a microorganism of the genus *Aeromonas* or a mutant of the polyhydroxyalkanoate synthase gene. More preferably, the gene (A) is a gene encoding an amino acid sequence having a sequence identity of 99.5 to 100% with an amino acid sequence of SEQ ID NO: 2 or 3.

Preferably, the gene (B) is composed of a combination of a part of a polyhydroxyalkanoate synthase gene derived from a microorganism of the genus *Aeromonas* and a part of a polyhydroxyalkanoate synthase gene derived from a microorganism of the genus *Cupriavidus.* More preferably, the gene (B) is a gene encoding an amino acid sequence having a sequence identity of 90 to 100% with an amino acid sequence of SEQ ID NO: 6.

Preferably, the gene (B) is a polyhydroxyalkanoate synthase gene derived from a microorganism of the genus *Chromobacterium* or a mutant of the polyhydroxyalkanoate synthase gene. More preferably, the gene (B) is a gene encoding an amino acid sequence having a sequence identity of 90 to 100% with an amino acid sequence of SEQ ID NO: 4 or 5.

Preferably, the gene (B) is a polyhydroxyalkanoate synthase gene derived from a microorganism of the genus *Bacillus* or a mutant of the polyhydroxyalkanoate synthase gene. More preferably, the gene (B) is a gene encoding amino acid sequences having a sequence identity of 90 to 100% with amino acid sequences of SEQ ID NOS: 7 and 8.

Preferably, the microorganism is a transformed microorganism that has been transformed to supply a greater amount of 3-hydroxyhexanoyl-CoA to an intracellular polyhydroxyalkanoate synthase than a wild strain of the microorganism. More preferably, the transformed microorganism has been transformed to inhibit degradation of an intermediate metabolite having six carbon atoms in β-oxidation of an oil or a fatty acid. Even more preferably, the transformed microorganism has been transformed to inhibit expression of a gene encoding a β-ketothiolase enzyme having thiolysis activity for β-ketohexanoyl-CoA which is β-ketoacyl-CoA having six carbon atoms.

Preferably, the β-ketothiolase enzyme has an amino acid sequence having a sequence identity of 90 to 100% with an amino acid sequence of SEQ ID NO: 9 or 10.

Preferably, the microorganism has a gene encoding a protein having (R)-specific enoyl-CoA hydratase activity.

Preferably, in the culturing step, a carbon source containing an oil or a fatty acid is added. More preferably, the carbon source containing an oil or a fatty acid is a carbon source containing a middle-chain fatty acid having 6 to 12 carbon atoms or a glyceride of the middle-chain fatty acid. Even more preferably, the middle-chain fatty acid is hexanoic acid.

Preferably, the microorganism belongs to the genus *Cupriavidus* or is a result of transformation of a microorganism of the genus *Cupriavidus.* More preferably, the microorganism is *Cupriavidus necator* or transformed *Cupriavidus necator.*

The present invention further relates to a transformed microorganism that produces a polyhydroxyalkanoate copolymer mixture, the transformed microorganism containing a gene encoding two types of polyhydroxyalkanoate synthases differing in polymerization activity for 3-hydroxyhexanoyl-CoA, wherein the transformed microorganism has been transformed to supply a greater amount of 3-hydroxyhexanoyl-CoA to an intracellular polyhydroxyalkanoate synthase than a wild strain of the transformed microorganism, the polyhydroxyalkanoate copolymer mixture contains: a polyhydroxyalkanoate fraction (I) containing a polyhydroxyalkanoate copolymer having a 3-hydroxybutyrate structural unit and a 3-hydroxyhexanoate structural unit, the polyhydroxyalkanoate fraction (I) having an average 3-hydroxyhexanoate unit ratio of 20 mol% or more; and a polyhydroxyalkanoate fraction (II) containing a polyhydroxyalkanoate having a 3-hydroxybutyrate structural unit, the polyhydroxyalkanoate fraction (II) having an average 3-hydroxyhexanoate unit ratio of 0 to 15 mol%, and the polyhydroxyalkanoate copolymer mixture has an average 3-hydroxyhexanoate unit ratio of 22 mol% or less.

### Advantageous Effects of Invention

The present invention makes it possible to produce a polyhydroxyalkanoate copolymer mixture having both excellent processability and excellent mechanical properties. According to a preferred embodiment of the present invention, the polyhydroxyalkanoate copolymer mixture produced is easy to handle, and industrial isolation and purification of the polyhydroxyalkanoate copolymer mixture from the microorganism can be easily carried out.

### Description of Embodiments

Hereinafter, an embodiment of the present invention will be described. The present invention is not limited to the embodiment described below.

The present invention is directed to a method of producing a PHA copolymer mixture, the method including the step of culturing a microorganism that produces the PHA copolymer mixture.

### (PHA Copolymer Mixture)

The PHA copolymer mixture is composed of: a PHA fraction (I) containing a PHA copolymer having a 3HB structural unit and a 3HH structural unit, the PHA fraction (I) having an average 3HH unit ratio of 20 mol% or more; and a PHA fraction (II) containing a PHA having a 3HB structural unit, the PHA fraction (II) having an average 3HH unit ratio of 0 to 15 mol%. The PHA copolymer mixture can be fractionated into the PHA fractions (1) and (II) by MIBK fractionation described later.

The PHA fraction (I) is a fraction containing a PHA copolymer having at least a 3HB structural unit and a 3HH structural unit, and may contain a PHA having a hydroxyalkanoate structural unit other than the 3HB and 3HH structural units. Preferably, the PHA fraction (I) is a fraction that contains a PHA copolymer having only the 3HB and 3HH structural units and not having any hydroxyalkanoate structural unit other than the 3HB and 3HH structural units. That is, the PHA fraction (I) is preferably a fraction that contains P(3HB-co-3HH).

Examples of the hydroxyalkanoate structural unit other than the 3HB and 3HH structural units include, but are not limited to, structural units derived from hydroxyalkanoates such as 3-hydroxypropionate, 3HV, 3-hydroxyalkanoates having 7 to 16 carbon atoms, 2-hydroxyalkanoates having 4 to 16 carbon atoms, 4-hydroxyalkanoates (e.g., 4-hydroxybutyrate), 5-hydroxyalkanoates, 6-hydroxyalkanoates (e.g., 6-hydroxyhexanoate), and lactic acid.

The average 3HH unit ratio of the PHA fraction (I) is 20 mol% or more, preferably 22 mol% or more, and more preferably 24 mol% or more. The upper limit of the average 3HH unit ratio is not limited to a particular value, and the average 3HH unit ratio is preferably 35 mol% or less, more preferably 32 mol% or less, and particularly preferably 30 mol% or less.

The PHA fraction (II) is a fraction containing a PHA having a 3HB structural unit. The PHA contained in the PHA fraction (II) may be a homopolymer having only the 3HB structural unit or a PHA copolymer having the 3HB structural unit and another hydroxyalkanoate structural unit. In view of the mechanical properties, the PHA is preferably a PHA copolymer having the 3HB structural unit and another hydroxyalkanoate structural unit. The PHA copolymer is preferably a PHA copolymer having the 3HB structural unit and further having a 3HV structural unit and/or a 3HH structural unit, more preferably a PHA copolymer having the 3HB and 3HH structural units, and even more preferably a PHA copolymer having only the 3HB and 3HH structural units, i.e., P(3HB-co-3HH).

The average 3HH unit ratio of the PHA fraction (II) is from 0 to 15 mol%. As for the lower limit, the average 3HH unit ratio is preferably 0.1 mol% or more, more preferably 1 mol% or more, even more preferably 2 mol% or more, and still even more preferably 3 mol% or more. As for the upper limit, the average 3HH unit ratio is preferably 12 mol% or less and more preferably 10 mol% or less.

The weight percentage of the PHA fraction (I) in the PHA copolymer mixture is preferably from 10 to 90%, more preferably from 20 to 80%, and particularly preferably from 30 to 70%. The weight percentage of the PHA fraction (II) in the PHA copolymer mixture is preferably from 10 to 90%, more preferably from 20 to 80%, and particularly preferably from 30 to 70%.

The average 3HH unit ratio of the entire PHA copolymer mixture is 22 mol% or less. If the average 3HH unit ratio is more than 22 mol%, the stickiness of the PHA copolymer mixture is so high that, in the step of isolating and purifying the PHA copolymer mixture after production by microbial fermentation, problems are likely to occur such as undesired formation of agglomerates and adhesion of the mixture to piping or pump equipment or clogging of the piping or pump equipment with the mixture, and industrial isolation and purification of the PHA copolymer mixture tends to be difficult.

In view of the balance between the mechanical properties of a molded article formed from the mixture and the processability and industrial handleability of the mixture, the average 3HH unit ratio of the entire PHA copolymer mixture is preferably from 10 to 22 mol%, more preferably from 11 to 20 mol%, even more preferably from 12 to 18 mol%, and particularly preferably from 13 to 17 mol%.

### (MIBK Fractionation)

The PHA copolymer mixture can be fractionated into the PHA fraction (I) having a higher average 3HH unit ratio and the PHA fraction (II) having a lower average 3HH unit ratio by solvent fractionation which makes use of the difference in solubility in methyl isobutyl ketone (MIBK). The higher the 3HH unit ratio of a PHA is, the higher the solubility of the PHA in MIBK is. Thus, the PHA copolymer mixture can be fractionated into the PHA fractions (I) and (II) by dissolving all of the PHA copolymer mixture in high-temperature MIBK and then lowering the temperature of the solution to precipitate the PHA component having a lower 3HH unit ratio.

The detailed procedures of the fractionation will now be described. First, about 100 mg of the PHA copolymer mixture is weighed into a screw-top test tube, to which 10 ml of MIBK is added and which is then closed with a cap. Subsequently, the test tube is heated at 140°C for about 1 to 3 hours under shaking to dissolve the PHA copolymer mixture completely. After the complete dissolution, the test tube is left at 25°C for 1 minute to cool the solution to a temperature lower than the boiling point. All of the cooled solution is quickly transferred to a centrifuge tube whose weight is already measured, and the centrifuge tube is closed with a cap. The centrifuge tube closed with the cap is left at 25°C for 15 minutes to precipitate a part of the dissolved matter. The precipitation is followed by centrifugation (9000 rpm, 5 minutes) to separate the precipitate and the solution from each other, and all of the solution is transferred to an aluminum cup whose weight is already measured. To the centrifuge tube containing the precipitate was added 10 ml of MIBK, and the contents of the tube are mixed by means of a vortex mixer and subjected to centrifugation (9000 rpm, 5 minutes). The separated solution is transferred to the aluminum cup containing the previously transferred solution. The aluminum cup is heated at 120°C for 30 minutes to evaporate MIBK and precipitate the dissolved matter. Further, the precipitate remaining in the aluminum cup and the precipitate remaining in the centrifuge tube are individually vacuum-dried at 100°C for 6 hours. The precipitate remaining in the aluminum cup is weighed as the PHA fraction (I), and the precipitate remaining in the centrifuge tube is weighed as the PHA fraction (II). The weighing is followed by confirming that the difference between the total weight of the PHA fractions (I) and (II) and the initially measured weight of the PHA copolymer mixture is within ±3%.

### (Melting Behavior of PHA Copolymer Mixture)

The PHA copolymer mixture preferably exhibits a highest melting peak temperature of 130°C or higher when subjected to differential scanning calorimetry. The PHA copolymer mixture that meets this requirement can be crystallized and solidified in a short time and thus has good processability. The highest melting peak temperature is preferably from 130 to 165°C and more preferably from 130 to 155°C.

The highest melting peak temperature exhibited by the PHA copolymer is measured as the highest of temperatures at which melting peaks appear in a DSC curve obtained by differential scanning calorimetry which uses a differential scanning calorimeter and in which the PHA copolymer mixture weighed to about 2 mg is heated from -30 to 200°C at a rate of 10°C/min.

The PHA copolymer mixture may exhibit, in addition to the highest-temperature melting peak, another melting peak in a temperature range below the temperature at which the highest-temperature melting peak is located. For example, the PHA copolymer mixture may exhibit a melting peak at a temperature of 100°C or lower.

### (PHA Copolymer Mixture-Producing Microorganism)

The microorganism used to produce the PHA copolymer mixture (this microorganism may be referred to as "PHA copolymer mixture-producing microorganism" hereinafter) is not limited to a particular type and may be any microorganism capable of fermentative production of the PHA copolymer mixture. The microorganism may be a wild strain that inherently accumulates PHAs, a mutant strain obtained by artificially mutating the wild strain, or a strain having a PHA accumulating ability attributed to a foreign PHA synthase gene introduced by a genetic engineering technique.

Preferred examples of the PHA copolymer mixture-producing microorganism or the host of a transformed microorganism serving as the PHA copolymer mixture-producing microorganism include, but are not limited to, bacteria belonging to the genus *Ralstonia,* the genus *Cupriavidus,* the genus *Wautersia*, the genus *Aeromonas,* the genus *Escherichia,* the genus *Alcaligenes,* and the genus *Pseudomonas.* In view of safety and PHA productivity, bacteria belonging to the genus *Ralstonia,* the genus *Cupriavidus,* the genus *Aeromonas,* and the genus *Wautersia* are more preferred. Even more preferred are bacteria belonging to the genus *Cupriavidus* and the genus *Aeromonas.* Still even more preferred are bacteria belonging to the genus *Cupriavidus.* Particularly preferred is *Cupriavidus necator.*

To efficiently produce two types of PHAs differing in average 3HH unit ratio, the PHA copolymer mixture-producing microorganism is preferably a microorganism having genes encoding two types of PHA synthases differing in polymerization activity for 3-hydroxyhexanoyl-CoA. 3-Hydroxyhexanoyl-CoA is a precursor of the 3HH structural unit contained in the PHA. When the microorganism has genes encoding two types of PHA synthases differing in polymerization activity for 3-hydroxyhexanoyl-CoA, a mixture of two types of PHAs differing significantly in average 3HH unit ratio, i.e., the PHA copolymer mixture, can be produced in the cells of the microorganism by fermentation. The microorganism has at least two types of genes encoding PHA synthases differing in polymerization activity for 3-hydroxyhexanoyl-CoA, and may have three or more types of such genes insofar as the microorganism is capable of fermentative production of the PHA copolymer mixture.

The two types of PHA synthases differing in polymerization activity for 3-hydroxyhexanoyl-CoA are not limited to a particular combination. Preferably, the amino acid sequence identity between the two types of PHA synthases is 90% or less. The amino acid sequence identity is more preferably 80% or less and even more preferably 70% or less. In general, PHA synthases are believed to form multimers such as dimers when performing their function. If the amino acid sequence identity between the two types of PHA synthases is more than 90%, the two types of PHA synthases could form hetero-dimers and fail to produce the PHA copolymer mixture.

Specific examples of the combination of the two types of PHA synthases differing in polymerization activity for 3-hydroxyhexanoyl-CoA include a combination of a gene (A) encoding a PHA synthase having higher polymerization activity for 3-hydroxyhexanoyl-CoA than an *Aeromonas caviae*-derived wild-type PHA synthase having the amino acid sequence of SEQ ID NO: 1 and a gene (B) encoding a PHA synthase having lower polymerization activity for 3-hydroxyhexanoyl-CoA than the *Aeromonas caviae*-derived wild-type PHA synthase.

Examples of the gene (A) include a polyhydroxyalkanoate synthase gene derived from a microorganism of the genus *Aeromonas* and a mutant of the polyhydroxyalkanoate synthase gene, and specific examples include a gene encoding an amino acid sequence having a sequence identity of 90 to 100% with the amino acid sequence of SEQ ID NO: 2 or 3 (the amino acid sequence of a PHA synthase mutant derived from a bacterium of the genus *Aeromonas*)*.* The sequence identity is preferably 95% or more, more preferably 97% or more, particularly preferably 99% or more, and most preferably 99.5% or more.

Examples of the gene (B) include a gene composed of a combination of a part of a polyhydroxyalkanoate synthase gene derived from a microorganism of the genus *Aeromonas* and a part of a polyhydroxyalkanoate synthase gene derived from a microorganism of the genus *Cupriavidus,* and specific examples include a gene encoding an amino acid sequence having a sequence identity of 90 to 100% with the amino acid sequence of SEQ ID NO: 6. Examples of the gene (B) further include a PHA synthase gene derived from a bacterium of the genus *Chromobacterium* and a mutant of the PHA synthase gene, and specific examples include a gene encoding an amino acid sequence having a sequence identity of 90 to 100% with the amino acid sequence of SEQ ID NO: 4 or 5. Examples of the gene (B) further include a PHA synthase gene derived from a bacterium of the genus *Bacillus* and a mutant of the PHA synthase gene, and specific examples include a gene encoding amino acid sequences having a sequence identity of 90 to 100% with the amino acid sequences of SEQ ID NOS: 7 and 8. The sequence identities as mentioned for the gene (B) are preferably 95% or more, more preferably 97% or more, and particularly preferably 99% or more.

To efficiently produce a PHA copolymer mixture having a high average 3HH unit ratio, the PHA copolymer mixture-producing microorganism is preferably a transformed microorganism that has been transformed to supply a greater amount of 3-hydroxyhexanoyl-CoA to an intracellular PHA synthase than a wild strain of the microorganism. Specifically, a transformed microorganism is preferred which has been transformed to inhibit degradation of an intermediate metabolite having six carbon atoms in β-oxidation of an oil or a fatty acid. The inhibition of the degradation of the intermediate metabolite having six carbon atoms in β -oxidation is inferred to result in an increase in the supply of 3-hydroxyhexanoyl-CoA and hence an increase in the average 3HH unit ratio of the PHA copolymer mixture produced.

Examples of the transformed microorganism that has been transformed to inhibit degradation of an intermediate metabolite having six carbon atoms in β-oxidation of an oil or a fatty acid include a transformed microorganism as described in Patent Literature 7 which has been transformed to inhibit the expression of a gene encoding a β-ketothiolase enzyme having thiolysis activity for β-ketohexanoyl-CoA which is β-ketoacyl-CoA having six carbon atoms. Examples of the β-ketothiolase enzyme-encoding gene include, but are not limited to, a gene encoding a β-ketothiolase enzyme having an amino acid sequence having a sequence identity of 90 to 100% with the amino acid sequence of SEQ ID NO: 9 or 10. The sequence identity is preferably 95% or more, more preferably 97% or more, and particularly preferably 99% or more.

Examples of methods of inhibiting the expression of the β-ketothiolase enzyme-encoding gene include: a method in which the enzyme gene is completely deleted from the transformed microorganism; a method in which a quite different gene such as a drug-resistant gene is inserted into the sequence of the enzyme gene; and a method in which a part of the sequence of the enzyme gene (the part is preferably a domain responsible for the enzyme activity) is mutated by deletion, substitution, addition, or insertion. Examples of the gene disruption process include a homologous recombination technique using a vector containing a gene or DNA for disruption and a technique using a transposon. Examples of other disruption methods include known techniques such as genome editing using a CRISPR/Cas (e.g., Cas9) system or TALEN for disrupting the target gene (Y. Wang et al., ACS Synth Biol. 2016, 5(7): 721-732; Bogdanove and Voytas, Science, 333: 1843-1846, 2011; Jinek et al., Science, 337: 816-821, 2012; Shalem et al., Science, 343: 84-87, 2014; and Wang et al., Science, 343: 80-84, 2014). For example, in the case of the CRISPR/Cas9 system, the guide RNA (gRNA) has a sequence capable of binding to a part of the base sequence of the β-ketothiolase gene to be disrupted and serves to carry the Cas9 to the target. Alternatively, a base sequence neighboring the target gene may be mutated by deletion, substitution, addition, or insertion to reduce the transcription-translation efficiency of the gene or the stability of the mRNA and thereby eliminate or reduce the enzyme activity.

To efficiently produce a PHA copolymer mixture having a high average 3HH unit ratio, the PHA copolymer mixture-producing microorganism is preferably a microorganism having a gene encoding a protein having (R)-specific enoyl-CoA hydratase activity. The (R)-specific enoyl-CoA hydratase has the function of converting hexenoyl-CoA to 3-hydroxyhexanoyl-CoA in microbial cells. Thus, when the microorganism has a gene encoding a protein having (R)-specific enoyl-CoA hydratase activity, the presence of the gene is inferred to enhance the conversion to 3-hydroxyhexanoyl-CoA and result in an increase in the average 3HH unit ratio of the PHA copolymer mixture produced.

The microorganism having a gene encoding a protein having (R)-specific enoyl-CoA hydratase activity may be a microorganism that inherently has the gene or a microorganism into which a foreign gene of this type has been introduced by a genetic engineering technique.

Examples of the foreign gene encoding a protein having (R)-specific enoyl-CoA hydratase activity include, but are not limited to: an *Aeromonas caviae*-derived gene encoding an (R)-specific enoyl-CoA hydratase having the amino acid sequence of SEQ ID NO: 11; a *Cupriavidus necator*-derived gene encoding (R)-specific enoyl-CoA hydratase having the amino acid sequence of SEQ ID NO: 12 or 13; a *Yarrowia lipolytica*-derived multifunctional enzyme type 2 (MFE2) gene encoding an enzyme having the amino acid sequence of SEQ ID NO: 14; a *Drosophila melanogaster-*derived MFE2 gene encoding an enzyme having the amino acid sequence of SEQ ID NO: 15; and a gene encoding a protein having (R)-specific enoyl-CoA hydratase activity and having a sequence identity of 85% or more, preferably 90% or more, more preferably 95% or more, particularly preferably 99% or more, with any of the amino acid sequences of SEQ ID NOS: 11 to 15.

To enhance the expression of the gene encoding a protein having (R)-specific enoyl-CoA hydratase activity, an expression regulatory sequence (promoter sequence and/or SD sequence) for enhancing the expression of the gene may be altered as described, for example, in WO 2015/115619.

In the case where the PHA copolymer mixture-producing microorganism has an introduced foreign gene, the introduced gene may be present on a chromosome possessed by the microorganism used as the host or on DNA such as a plasmid or megaplasmid possessed by the microorganism used as the host. In view of holding of the introduced gene, the introduced gene is preferably present on a chromosome or megaplasmid possessed by the microorganism and more preferably present on a chromosome possessed by the microorganism. In the case of enhancing the expression of a gene inherently possessed by the microorganism used as the host, a base sequence upstream of the gene may be mutated by substitution, deletion, or addition to enhance the expression of the gene.

Methods of modifying DNA of a microorganism by site-specific substitution with or insertion of given DNA or deleting a given site of the DNA of the microorganism are known to those skilled in the art, and any of the known methods can be used to produce the transformed microorganism according to the present embodiment. Typical examples of the methods include, but are not limited to: a method using a transposon and the mechanism of homologous recombination (Ohman et al., J. Bacteriol., vol. 162: p. 1068 (1985)); a method based on site-specific integration induced by the mechanism of homologous recombination and on loss due to second homologous recombination (Noti et al., Methods Enzymol., vol. 154, p. 197 (1987)); and a method in which a sacB gene derived from *Bacillus subtilis* is allowed to coexist and thus in which a microbial strain having lost a gene due to second homologous recombination is easily isolated as a sucrose-supplemented medium-resistant strain (Schweizer, Mol. Microbiol., vol. 6, p. 1195 (1992); Lenz et al., J. Bacteriol., vol. 176, p. 4385 (1994)). The method of introducing a vector into cells is not limited to a particular technique, and examples of the method include calcium chloride transformation, electroporation, polyethylene glycol transformation, and spheroplast transformation.

For gene cloning or gene recombination, a technique as described in Sambrook, J. et al., Molecular Cloning: A Laboratory Manual, Cold Spring Harbor Laboratory Press (1989 or 2001) can be used.

The promoter for expressing the introduced gene is not limited to a particular type. Examples of usable promoters include: a phaC1 gene promoter and a phaP1 gene promoter of *Cupriavidus necator;* a lac promoter, a lacUV5 promoter, a trc promoter, a tic promoter, and a tac promoter which are derived from *Escherichia coli;* an artificially prepared lacN17 promoter having an *Escherichia coli*-derived altered base sequence represented by SEQ ID NO: 16; and an artificially prepared lacN19 promoter having an *Escherichia coli*-derived altered base sequence represented by SEQ ID NO: 17.

### (Culture of Microorganism)

Culturing the PHA copolymer mixture-producing microorganism allows the microbial cells to accumulate the PHA copolymer mixture therein. The culture of the PHA copolymer mixture-producing microorganism can be conducted by a common microbial culture method, and it is sufficient that the PHA copolymer mixture-producing microorganism be cultured in a culture medium containing a suitable carbon source. There are no particular limitations on the composition of the culture medium, the method of adding the carbon source, the scale of the culture, the conditions of aeration and stirring, the culture temperature, and the culture time. It is preferable to add the carbon source to the culture medium continuously or intermittently.

The carbon source used for the culture may be any carbon source that can be assimilated by the PHA copolymer mixture-producing microorganism. Examples of the carbon source include, but are not limited to: sugars such as glucose, fructose, sucrose, and xylose; oils such as palm and palm kernel oils (including palm olein, palm double olein, and palm kernel olein which are low-melting fractions obtained through fractionation of palm oil and palm kernel oil), corn oil, coconut oil, olive oil, soybean oil, rapeseed oil, and Jatropha oil; fractions of these oils; by-products formed during refining of these oils; fatty acids such as lauric acid, oleic acid, stearic acid, palmitic acid, and myristic acid; derivatives of these fatty acids; and glycerol. In the case where the PHA copolymer mixture-producing microorganism can assimilate gases such as carbon dioxide, carbon monoxide, and methane or alcohols such as methanol and ethanol, any of these gases or alcohols can be used as the carbon source.

In particular, the carbon source preferably contains an oil or a fatty acid. The oil is preferably a vegetable oil or a fraction thereof. The chain length of the oil or fatty acid is preferably short. The carbon source more preferably contains a medium-chain fatty acid having 6 to 12 carbon atoms or a glyceride of the medium-chain fatty acid and even more preferably contains hexanoic acid. When the number of carbon atoms in the fatty acid contained in the carbon source is from 6 to 12, it is expected that an increased amount of intermediate metabolite having six carbon atoms is produced in β-oxidation and hence that a PHA fraction having a high average 3HH unit ratio is efficiently obtained.

In the production of the PHA copolymer mixture, it is preferable to culture the microorganism using a culture medium containing the carbon source as described above and other nutrient sources including a nitrogen source, an inorganic salt, and another organic nutrient source. Examples of the nitrogen source include, but are not limited to: ammonia; ammonium salts such as ammonium chloride, ammonium sulfate, and ammonium phosphate; peptone; meat extracts; and yeast extracts. Examples of the inorganic salt include potassium dihydrogen phosphate, sodium dihydrogen phosphate, magnesium phosphate, magnesium sulfate, and sodium chloride. Examples of the other organic nutrient source include: amino acids such as glycine, alanine, serine, threonine, and proline; and vitamins such as vitamin B1, vitamin B 12, and vitamin C.

After the PHA copolymer mixture-producing microorganism is cultured for an adequate time to allow the microbial cells to accumulate the PHA copolymer mixture therein, the PHA copolymer mixture is collected from the microbial cells using a known method. The method of collection is not limited to a particular technique. From the industrial point of view, it is preferable to collect the PHA copolymer mixture by separation and purification using an aqueous system which has a low environmental impact. For example, the cultured cells may be disrupted by application of a mechanical shear force or by the use of a surfactant, an alkali, or an enzyme to obtain a cell disruption solution in which cellular components other than the PHAs are dissolved in water. The PHA copolymer mixture can be collected by separating the PHA copolymer mixture from the aqueous phase through filtration or centrifugation of the cell disruption solution and then drying the separated PHA copolymer mixture.

### Examples

Hereinafter, the present invention will be described in more detail using examples. The present invention is not limited to the examples. The overall genetic manipulation can be carried out, for example, in a manner as taught in Molecular Cloning (Cold Spring Harbor Laboratory Press (1989)). The enzymes, cloning hosts, and other materials used in the gene manipulation can be purchased from market suppliers and used according to the instructions given by the suppliers. The enzymes are not limited to particular types and may be any enzymes that can be used for gene manipulation.

### (Microbial Strain Preparation Example 1)

### Preparation of PHA copolymer mixture-producing microbial strain (1)

First, a plasmid for PHA synthase gene disruption was prepared. The preparation was done as follows.

PCR using synthetic oligo DNA was carried out to obtain a DNA fragment (SEQ ID NO: 18) having base sequences upstream and downstream of the phaC1 structural gene (PHA synthase gene) of *Cupriavidus necator* H16. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with Swal. In this manner, a plasmid vector pNS2X-sacB+phaC1UD for PHA synthase gene disruption was prepared.

Subsequently, a PHA synthase gene-disrupted strain was prepared using the plasmid vector pNS2X-sacB+phaC1UD for PHA synthase gene disruption. The preparation was done as follows.

*Escherichia coli* S17-1 (ATCC 47055) was transformed with the plasmid vector pNS2X-sacB+phaC1UD for PHA synthase gene disruption, and the resulting transformed microorganism was co-cultured with KNK005dZ/trc-J4b/dbktB/dA1528 on Nutrient Agar (manufactured by Difco Laboratories) to effect conjugal transfer.

The KNK005dZ/trc-J4b/dbktB/dA1528 is a strain in which: the phaZ1, phaZ2, and phaZ6 genes on the chromosome of *Cupriavidus necator* H16 are deleted; the PHA synthase gene on the chromosome is substituted with an altered PHA synthase gene derived from *Aeromonas caviae* (a gene encoding a PHA synthase having the amino acid sequence of SEQ ID NO: 2, i.e., N149S/D171 G mutant gene); the expression of the (R)-specific enoyl-CoA hydratase gene on the chromosome is enhanced; and the bktB and A1528 structural genes are deleted. This strain can be prepared according to the method described in WO 2019/142845.

The culture fluid obtained as above was inoculated into a Simmons agar medium (2 g/L sodium citrate, 5 g/L sodium chloride, 0.2 g/L magnesium sulfate heptahydrate, 1 g/L ammonium dihydrogen phosphate, 1 g/L potassium dihydrogen phosphate, 15 g/L agar, pH = 6.8) containing 250 mg/L kanamycin, and strains grown on the agar medium were selectively collected. Thus, a strain having the plasmid integrated into the chromosome of the KNK005dZ/trc-J4b/dbktB/dA1528 was obtained. The obtained strain was cultured in Nutrient Broth (manufactured by Difco Laboratories) for two generations, after which the culture broth was diluted and applied onto Nutrient Agar containing 15% sucrose. Strains grown on Nutrient Agar were obtained as strains having lost the plasmid. PCR and analysis using a DNA sequencer were further carried out to isolate one strain having a chromosome from which the PHA synthase gene was deleted. This gene-disrupted strain was named "KNK005dZ/dNSDG/trc-J4b/dbktB/dA1528".

A plasmid for PHA synthase gene introduction was also prepared. The preparation was done as follows.

PCR using synthetic oligo DNA was carried out to obtain a DNA fragment (SEQ ID NO: 19) having base sequences upstream and downstream of the bktB structural gene (β-ketothiolase gene) of *Cupriavidus necator* H16, having a lacN19 promoter which is an altered lac promoter of *Escherichia coli,* and having a base sequence of a gene encoding a PHA synthase having the amino acid sequence of SEQ ID NO: 2. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+bktbU-lacN19-NSDG-bktbD for PHA synthase gene introduction was prepared.

Next, a PHA synthase gene-introduced strain was prepared using the plasmid vector pNS2X-sacB+bktbU-lacN19-NSDG-bktbD for PHA synthase gene introduction. The preparation was done as follows.

The plasmid vector pNS2X-sacB+bktbU-lacN19-NSDG-bktbD for PHA synthase gene introduction was introduced into the KNK005dZ/dNSDG/trc-J4b/dbktB/dA1528 by procedures using conjugal transfer as described above. Further, culture and selection on Nutrient Agar containing 15% sucrose were carried out as described above to isolate one strain having a chromosome on which the lacN19 promoter and the gene encoding the PHA synthase having the amino acid sequence of SEQ ID NO: 2 were introduced at a position where the bktB gene was originally present. The strain thus obtained was named "KNK005dZ/dNSDG/trc-J4b/dbktB: :lacN19-NSDG/dA1528".

A plasmid for PHA synthase gene introduction was further prepared. The preparation was done as follows.

PCR using synthetic oligo DNA was carried out to obtain a DNA fragment (SEQ ID NO: 20) having base sequences upstream and downstream of the A1528 structural gene (β-ketothiolase gene) of *Cupriavidus necator* H16, having a lacN17 promoter which is an altered lac promoter of *Escherichia coli,* and having a base sequence of a gene encoding a PHA synthase having the amino acid sequence of SEQ ID NO: 6. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+A1528U-lacN17-AcNSRe12-A1528D for PHA synthase gene introduction was prepared.

Next, the plasmid vector pNS2X-sacB+A1528U-lacN17-AcNSRe12-A1528D for PHA synthase gene introduction was introduced into the KNK005dZ/dNSDG/trc-J4b/dbktB::lacN19-NSDG/dA1528 by procedures using conjugal transfer as described above. Further, culture and selection on Nutrient Agar containing 15% sucrose were carried out as described above to isolate one strain having a chromosome on which the lacN17 promoter and the gene encoding the PHA synthase having the amino acid sequence of SEQ ID NO: 6 were introduced at a position where the A1528 gene was originally present. The strain thus obtained was named "KNK005dZ/dNSDG/trc-J4b/dbktB::lacN19-NSDG/dA1528::lacN17-AcNSRe12" (also referred to as "PHA copolymer mixture-producing microbial strain (1)" hereinafter).

The PHA copolymer mixture-producing microbial strain (1) is a strain in which: the phaZ1, phaZ2, and phaZ6 genes on the chromosome of *Cupriavidus necator* H16 are deleted; a gene encoding an *Aeromonas-*derived PHA synthase mutant having the amino acid sequence of SEQ ID NO: 2 and a gene encoding a PHA synthase having the amino acid sequence of SEQ ID NO: 6 (i.e., a PHA synthase-encoding gene composed of a combination of a part of a polyhydroxyalkanoate synthase gene derived from a microorganism of the genus *Aeromonas* and a part of a polyhydroxyalkanoate synthase gene derived from a microorganism of the genus *Cupriavidus*) are introduced; the expression of the (R)-specific enoyl-CoA hydratase gene on the chromosome is enhanced; and the bktB structural gene (β-ketothiolase gene) and the A1528 structural gene (β-ketothiolase gene) are deleted.

### (Microbial Strain Preparation Example 2)

### Preparation of PHA copolymer mixture-producing microbial strain (2)

First, a plasmid for PHA synthase gene introduction was prepared. The preparation was done as follows.

PCR using synthetic oligo DNA was carried out to obtain a DNA fragment (SEQ ID NO: 21) having base sequences upstream and downstream of the bktB structural gene (β-ketothiolase gene) of *Cupriavidus necator* H16, having a lac promoter of *Escherichia coli,* and having a base sequence of a gene encoding a PHA synthase having the amino acid sequence of SEQ ID NO: 2. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with Swal. In this manner, a plasmid vector pNS2X-sacB+bktbU-lac-NSDG-bktbD for PHA synthase gene introduction was prepared.

Next, a PHA synthase gene-introduced strain was prepared using the plasmid vector pNS2X-sacB+bktbU-lac-NSDG-bktbD for PHA synthase gene introduction. The preparation was done as follows.

The plasmid vector pNS2X-sacB+bktbU-lac-NSDG-bktbD for PHA synthase gene introduction was introduced into the KNK005dZ/dNSDG/trc-J4b/dbktB/dA1528 by procedures using conjugal transfer as described above. Further, culture and selection on Nutrient Agar containing 15% sucrose were carried out as described above to isolate one strain having a chromosome on which the lac promoter and the gene encoding the PHA synthase having the amino acid sequence of SEQ ID NO: 2 were introduced at a position where the bktB gene was originally present. The strain thus obtained was named "KNK005dZ/dNSDG/trc-J4b/dbktB::lac-NSDG/dA1528".

Next, the plasmid vector pNS2X-sacB+A1528U-lacN17-AcNSRe12-A1528D for PHA synthase gene introduction was introduced into the KNK005dZ/dNSDG/trc-J4b/dbktB::lac-NSDG/dA1528 by procedures using conjugal transfer as described above. Further, culture and selection on Nutrient Agar containing 15% sucrose were carried out as described above to isolate one strain having a chromosome on which the lacN17 promoter and the gene encoding the PHA synthase having the amino acid sequence of SEQ ID NO: 6 were introduced at a position where the A1528 gene was originally present. The strain thus obtained was named "KNK005dZ/dNSDG/trc-J4b/dbktB::lac-NSDG/dA1528::lacN17-AcNSRe12" (also referred to as "PHA copolymer mixture-producing microbial strain (2)" hereinafter).

The PHA copolymer mixture-producing microbial strain (2) is a strain in which: the phaZ1, phaZ2, and phaZ6 genes on the chromosome of *Cupriavidus necator* H16 are deleted; a gene encoding an *Aeromonas-*derived PHA synthase mutant having the amino acid sequence of SEQ ID NO: 2 and a gene encoding a PHA synthase having the amino acid sequence of SEQ ID NO: 6 (i.e., a PHA synthase-encoding gene composed of a combination of a part of a polyhydroxyalkanoate synthase gene derived from a microorganism of the genus *Aeromonas* and a part of a polyhydroxyalkanoate synthase gene derived from a microorganism of the genus *Cupriavidus)* are introduced; the expression of the (R)-specific enoyl-CoA hydratase gene on the chromosome is enhanced; and the bktB structural gene (β-ketothiolase gene) and the A1528 structural gene (β-ketothiolase gene) are deleted.

### (Microbial Strain Preparation Example 3)

### Preparation of PHA copolymer mixture-producing microbial strain (3)

First, a plasmid for PHA synthase gene expression was prepared. The preparation was done as follows. PCR using synthetic oligo DNA was carried out to obtain a DNA fragment (SEQ ID NO: 22) having a lacN17 promoter which is an altered lac promoter of *Escherichia coli.* The DNA fragment was digested with restriction enzymes EcoRI and MunI, and the resulting DNA fragment was joined to a plasmid vector pCUP2 which is described in WO 2007/049716 and which was cleaved with MunI. A fragment-vector conjugate was selected in which the DNA fragment was joined to pCUP2 in such an orientation that the restriction enzyme SpeI-recognition sequence of pCUP2 was located downstream of the lacN17 promoter. In this manner, pCUP2-lacN17 was obtained. Subsequently, PCR using synthetic oligo DNA was carried out to obtain a DNA fragment (SEQ ID NO: 23) having a base sequence of a gene encoding a PHA synthase having the amino acid sequence of SEQ ID NO: 6 and a base sequence of a gene encoding a PHA synthase having the amino acid sequence of SEQ ID NO: 2. The DNA fragment was digested with restriction enzymes MunI and SpeI, and the resulting DNA fragment was joined to pCUP2-lacN17 cleaved with MunI and Spel. In this manner, a plasmid pCUP2-lacN17-AcNSRe12-NSDG for PHA synthase gene expression was obtained.

Next, the plasmid pCUP2-lacN17-AcNSRe12-NSDG for PHA synthase gene expression was introduced into the KNK005dZ/dNSDG/trc-J4b/dbktB/dA1528 to obtain pCUP2-lacN17-AcNSRe12-NSDG/KNK005dZ/dNSDG/trc-J4b/dbktB/dA1528 (also referred to as "PHA copolymer mixture-producing microbial strain (3)" hereinafter).

The introduction of the plasmid vector into the cells was carried out by electroporation as described below. The gene introduction device used was Gene Pulser manufactured by Bio-Rad Laboratories, Inc., and the cuvette used was a 0.2-cm-gap cuvette also manufactured by Bio-Rad Laboratories, Inc. The cuvette was charged with 400 µl of competent cells and 20 µl of the expression vector and set on the pulse device, by which electric pulse was applied to the contents of the cuvette at a capacitance of 25 µF, a voltage of 1.5 kV, and a resistance value of 800 Ω. After the pulse application, the cell-containing fluid in the cuvette was subjected to shake culture in Nutrient Broth (manufactured by Difco Laboratories) at 30°C for 3 hours and then to culture on a selection plate (Nutrient Agar manufactured by Difco Laboratories, containing 100 mg/L kanamycin) at 30°C for 2 days. The PHA copolymer mixture-producing microbial strain (3) thus grown was collected.

The PHA copolymer mixture-producing microbial strain (3) is a strain in which: the phaZ1, phaZ2, and phaZ6 genes on the chromosome of *Cupriavidus necator* H16 are deleted; a gene encoding an *Aeromonas-*derived PHA synthase mutant having the amino acid sequence of SEQ ID NO: 2 and a gene encoding a PHA synthase having the amino acid sequence of SEQ ID NO: 6 (i.e., a PHA synthase-encoding gene composed of a combination of a part of a polyhydroxyalkanoate synthase gene derived from a microorganism of the genus *Aeromonas* and a part of a polyhydroxyalkanoate synthase gene derived from a microorganism of the genus *Cupriavidus)* are introduced; the expression of the (R)-specific enoyl-CoA hydratase gene on the chromosome is enhanced; and the bktB structural gene (β-ketothiolase gene) and the A1528 structural gene (β-ketothiolase gene) are deleted.

### (Microbial Strain Preparation Example 4)

### Preparation of PHA copolymer mixture-producing microbial strain (4)

First, a plasmid for PHA synthase gene introduction was prepared. The preparation was done as follows.

PCR using synthetic oligo DNA was carried out to obtain a DNA fragment (SEQ ID NO: 24) having base sequences upstream and downstream of the bktB structural gene (β-ketothiolase gene) of *Cupriavidus necator* H16, having a lac promoter of *Escherichia coli,* having a base sequence of a gene encoding a PHA synthase having the amino acid sequence of SEQ ID NO: 6, and having a base sequence of a gene encoding a PHA synthase having the amino acid sequence of SEQ ID NO: 2. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+bktbU-lac-AcNSRe12-NSDG-bktbD for PHA synthase gene introduction was prepared.

Next, a PHA synthase gene-introduced strain was prepared using the plasmid vector pNS2X-sacB+bktbU-lac-AcNSRe12-NSDG-bktbD for PHA synthase gene introduction. The preparation was done as follows.

The plasmid vector pNS2X-sacB+bktbU-lac-AcNSRe12-NSDG-bktbD for PHA synthase gene introduction was introduced into the KNK005dZ/dNSDG/trc-J4b/dbktB/dA1528 by procedures using conjugal transfer as described above. Further, culture and selection on Nutrient Agar containing 15% sucrose were carried out as described above to isolate one strain having a chromosome on which the lac promoter, the gene encoding the PHA synthase having the amino acid sequence of SEQ ID NO: 6, and the gene encoding the PHA synthase having the amino acid sequence of SEQ ID NO: 2 were introduced at a position where the bktB gene was originally present. The strain thus obtained was named "KNK005dZ/dNSDG/trc-J4b/dbktB::lac-AcNSRe12-NSDG/dA1528" (also referred to as "PHA copolymer mixture-producing microbial strain (4)" hereinafter).

The PHA copolymer mixture-producing microbial strain (4) is a strain in which: the phaZ1, phaZ2, and phaZ6 genes on the chromosome of *Cupriavidus necator* H16 are deleted; a gene encoding an *Aeromonas-*derived PHA synthase mutant having the amino acid sequence of SEQ ID NO: 2 and a gene encoding a PHA synthase having the amino acid sequence of SEQ ID NO: 6 (i.e., a PHA synthase-encoding gene composed of a combination of a part of a polyhydroxyalkanoate synthase gene derived from a microorganism of the genus *Aeromonas* and a part of a polyhydroxyalkanoate synthase gene derived from a microorganism of the genus *Cupriavidus)* are introduced; the expression of the (R)-specific enoyl-CoA hydratase gene on the chromosome is enhanced; and the bktB structural gene (β-ketothiolase gene) and the A1528 structural gene (β-ketothiolase gene) are deleted.

### (Microbial Strain Preparation Example 5)

### Preparation of PHA copolymer mixture-producing microbial strain (5)

First, a plasmid for PHA synthase gene expression was prepared. The preparation was done as follows. PCR using synthetic oligo DNA was carried out to obtain a DNA fragment (SEQ ID NO: 26) having a base sequence of a gene encoding a PHA synthase having the amino acid sequence of SEQ ID NO: 7, a base sequence of a gene encoding a PHA synthase having the amino acid sequence of SEQ ID NO: 8, and a base sequence of a gene encoding a PHA synthase having the amino acid sequence of SEQ ID NO: 3. The DNA fragment was digested with restriction enzymes MunI and SpeI, and the resulting DNA fragment was joined to pCUP2-lacN17 cleaved with MunI and SpeI. In this manner, a plasmid pCUP2-lacN17-RCYB4-NSDGST for PHA synthase gene expression was obtained.

Next, the plasmid pCUP2-lacN 17-RCYB4-NSDGST for PHA synthase gene expression was introduced into the KNK005dZ/dNSDG/trc-J4b/dbktB/dA1528 by electroporation as described in Microbial Strain Preparation Example 3 to obtain pCUP2-lacN17-RCYB4-NSDGST/KNK005dZ/dNSDG/trc-J4b/dbktB/dA1528 (also referred to as "PHA copolymer mixture-producing microbial strain (5)" hereinafter).

The PHA copolymer mixture-producing microbial strain (5) is a strain in which: the phaZ1, phaZ2, and phaZ6 genes on the chromosome of *Cupriavidus necator* H16 are deleted; a gene encoding an *Aeromonas-*derived PHA synthase mutant having the amino acid sequence of SEQ ID NO: 3 and a gene encoding a *Bacillus*-derived PHA synthase having the amino acid sequences of SEQ ID NOS: 7 and 8 are introduced; the expression of the (R)-specific enoyl-CoA hydratase gene on the chromosome is enhanced; and the bktB structural gene (β-ketothiolase gene) and the A1528 structural gene (β-ketothiolase gene) are deleted.

### (Microbial Strain Preparation Example 6)

### Preparation of PHA copolymer mixture-producing microbial strain (6)

First, a plasmid for PHA synthase gene introduction was prepared. The preparation was done as follows. PCR using synthetic oligo DNA was carried out to obtain a DNA fragment (SEQ ID NO: 27) having base sequences upstream and downstream of the A1528 structural gene (β-ketothiolase gene) of *Cupriavidus necator* H16, having a lacN17 promoter which is an altered lac promoter of *Escherichia coli,* and having a base sequence of a gene encoding a PHA synthase having the amino acid sequence of SEQ ID NO: 5. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+A1528U-lacN17-CsAG-A1528D for PHA synthase gene introduction was prepared.

The plasmid vector pNS2X-sacB+A1528U-lacN17-CsAG-A1528D for PHA synthase gene introduction was introduced into the KNK005dZ/dNSDG/trc-J4b/dbktB::lac-NSDG/dA1528 by procedures using conjugal transfer as described above. Further, culture and selection on Nutrient Agar containing 15% sucrose were carried out as described above to isolate one strain having a chromosome on which the lacN17 promoter and the gene encoding the PHA synthase having the amino acid sequence of SEQ ID NO: 5 were introduced at a position where the A1528 gene was originally present. The strain thus obtained was named "KNK005dZ/dNSDG/trc-J4b/dbktB::lac-NSDG/dA1528::lacN17-CsAG" (also referred to as "PHA copolymer mixture-producing microbial strain (6)" hereinafter).

The PHA copolymer mixture-producing microbial strain (6) is a strain in which: the phaZ1, phaZ2, and phaZ6 genes on the chromosome of *Cupriavidus necator* H16 are deleted; a gene encoding an *Aeromonas-*derived PHA synthase mutant having the amino acid sequence of SEQ ID NO: 2 and a gene encoding a *Chromobacterium*-derived PHA synthase having the amino acid sequence of SEQ ID NO: 5 are introduced; the expression of the (R)-specific enoyl-CoA hydratase gene on the chromosome is enhanced; and the bktB structural gene (β-ketothiolase gene) and the A1528 structural gene (β-ketothiolase gene) are deleted.

### (Microbial Strain Preparation Example 7)

### Preparation of P(3HB-co-3HH)-producing microbial strain (1)

KNK005dZ (also referred to as "P(3HB-co-3HH)-producing microbial strain (1)" hereinafter) is a transformed microorganism in which an *Aeromonas*-derived PHA synthase gene (a gene encoding a PHA synthase having the amino acid sequence of SEQ ID NO: 2) is introduced on the chromosome of *Cupriavidus necator* H16 and in which the phaZ1, phaZ2, and phaZ6 genes acting as PHA-degrading enzyme genes are deleted from the chromosome. This transformed microorganism can be prepared according to the method described in WO 2014/065253.

### (Microbial Strain Preparation Example 8)

### Preparation of P(3HB-co-3HH)-producing microbial strain (2)

First, a plasmid for PHA synthase gene introduction was prepared. The preparation was done as follows.

PCR using synthetic oligo DNA was carried out to obtain a DNA fragment (SEQ ID NO: 28) having base sequences upstream and downstream of the phaZ6 structural gene of *Cupriavidus necator* H16, having a lacN17 promoter which is an altered lac promoter of *Escherichia coli,* and having a base sequence of a gene encoding a PHA synthase having the amino acid sequence of SEQ ID NO: 2. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with Swal. In this manner, a plasmid vector pNS2X-sacB+phaZ6U-lacN17-NSDG-phaZ6D for PHA synthase gene introduction was prepared.

Next, the plasmid vector pNS2X-sacB+phaZ6U-lacN17-NSDG-phaZ6D for PHA synthase gene introduction was introduced into KNK005dZ/trc-J4b/dbktB by procedures using conjugal transfer as described above.

The KNK005dZ/trc-J4b/dbktB is a strain in which: the phaZ1, phaZ2, and phaZ6 genes on the chromosome of *Cupriavidus necator* H16 are deleted; the PHA synthase gene on the chromosome is substituted with an altered PHA synthase gene derived from *Aeromonas caviae* (a gene encoding a PHA synthase having the amino acid sequence of SEQ ID NO: 2, i.e., N149S/D171G mutant gene); the expression of the (R)-specific enoyl-CoA hydratase gene on the chromosome is enhanced; and the bktB structural gene is deleted. This strain can be prepared according to the method described in WO 2019/142845.

Further, culture and selection on Nutrient Agar containing 15% sucrose were carried out as described above to isolate one strain having a chromosome on which the lacN17 promoter and the gene encoding the PHA synthase having the amino acid sequence of SEQ ID NO: 2 were introduced at a position where the phaZ6 gene was originally present. The strain thus obtained was named "KNK005dZ/trc-J4b/Z6::lacN17-NSDG/dbktB" (also referred to as "P(3HB-co-3HH)-producing microbial strain (2)" hereinafter).

### (Microbial Strain Preparation Example 9)

### Preparation of P(3HB-co-3HH)-producing microbial strain (3)

First, the plasmid vector pNS2X-sacB+phaC1UD for PHA synthase gene disruption was introduced into the KNK005dZ/trc-J4b/dbktB by procedures using conjugal transfer as described above.

Further, culture and selection on Nutrient Agar containing 15% sucrose were carried out as described above to isolate one strain having a chromosome from which the PHA synthase gene was deleted. The strain thus obtained was named "KNK005dZ/dNSDG/trc-J4b/dbktB".

Next, a plasmid for PHA synthase gene introduction was prepared. The preparation was done as follows.

PCR using synthetic oligo DNA was carried out to obtain a DNA fragment (SEQ ID NO: 29) having base sequences upstream and downstream of the phaC1 structural gene of *Cupriavidus necator* H16 and having a base sequence of a gene encoding a PHA synthase having the amino acid sequence of SEQ ID NO: 3. The DNA fragment was digested with a restriction enzyme SwaI, and the resulting DNA fragment was joined by a DNA ligase (Ligation High, manufactured by Toyobo Co., Ltd.) to a vector pNS2X-sacB which is described in Japanese Laid-Open Patent Application Publication No. 2007-259708 and which was also digested with SwaI. In this manner, a plasmid vector pNS2X-sacB+phaC1U-NSDGST-phaC1D for PHA synthase gene introduction was prepared.

Next, the plasmid vector pNS2X-sacB+phaC1U-NSDGST-phaC1D for PHA synthase gene introduction was introduced into the KNK005dZ/dNSDG/trc-J4b/dbktB by procedures using conjugal transfer as described above.

Further, culture and selection on Nutrient Agar containing 15% sucrose were carried out as described above to isolate one strain having a chromosome on which the gene encoding the PHA synthase having the amino acid sequence of SEQ ID NO: 3 was introduced at a position where the phaC1 gene was originally present. The strain thus obtained was named "KNK005dZ/NSDGST/trc-J4b/dbktB".

Next, a plasmid for PHA synthase gene expression was prepared. The preparation was done as follows. PCR using synthetic oligo DNA was carried out to obtain a DNA fragment (SEQ ID NO: 30) having a base sequence of a gene encoding a PHA synthase having the amino acid sequence of SEQ ID NO: 3. The DNA fragment was digested with restriction enzymes MunI and SpeI, and the resulting DNA fragment was joined to pCUP2 which is described in WO 2007/049716 and which was cleaved with Munl and SpeI. In this manner, pCUP2-NSDGST was obtained. Subsequently, PCR using synthetic oligo DNA was carried out to obtain a DNA fragment (SEQ ID NO: 31) having a trp promoter. The DNA fragment was digested with a restriction enzyme MunI, and the resulting DNA fragment was joined to pCUP2-NSDGST cleaved with MunI in such an orientation that the PHA synthase-encoding gene was located downstream of the trp promoter. In this manner, a plasmid pCUP2-trp-NSDGST for PHA synthase gene expression was obtained.

Subsequently, the plasmid pCUP2-trp-NSDGST for PHA synthase gene expression was introduced into the KNK005dZ/NSDGST/trc-J4b/dbktB by electroporation as described in Microbial Strain Preparation Example 3 to obtain pCUP2-trp-NSDGST/KNK005dZ/NSDGST/trc-J4b/dbktB (also referred to as "P(3HB-co-3HH)-producing microbial strain (3)" hereinafter).

The P(3HB-co-3HH)-producing microbial strain (3) is a strain in which: the phaZ1, phaZ2, and phaZ6 genes on the chromosome of *Cupriavidus necator* H16 are deleted; a gene encoding an *Aeromonas-*derived PHA synthase mutant having the amino acid sequence of SEQ ID NO: 3 is introduced; the expression of the (R)-specific enoyl-CoA hydratase gene on the chromosome is enhanced; and the bktB structural gene (β-ketothiolase gene) is deleted.

### (Microbial Strain Preparation Example 10)

### Preparation of P(3HB-co-3HH)-producing microbial strain (4)

First, the plasmid vector pNS2X-sacB+phaC1U-NSDGST-phaClD for PHA synthase gene introduction was introduced into the KNK005dZ/dNSDG/trc-J4b/dbktB/dA1528 by procedures using conjugal transfer as described above.

Further, culture and selection on Nutrient Agar containing 15% sucrose were carried out as described above to isolate one strain having a chromosome on which the gene encoding the PHA synthase having the amino acid sequence of SEQ ID NO: 3 was introduced at a position where the phaC1 gene was originally present. The strain thus obtained was named "KNK005dZ/NSDGST/trc-J4b/dbktB/dA1528".

Next, the plasmid pCUP2-trp-NSDGST for PHA synthase gene expression was introduced into the KNK005dZ/NSDGST/trc-J4b/dbktB/dA1528 by electroporation as described in Microbial Strain Preparation Example 3 to obtain pCUP2-trp-NSDGST/KNK005dZ/NSDGST/trc-J4b/dbktB/dA1528 (also referred to as "P(3HB-co-3HH)-producing microbial strain (4)" hereinafter).

The P(3HB-co-3HH)-producing microbial strain (4) is a strain in which: the phaZ1, phaZ2, and phaZ6 genes on the chromosome of *Cupriavidus necator* H16 are deleted; a gene encoding an *Aeromonas-derived* PHA synthase mutant having the amino acid sequence of SEQ ID NO: 3 is introduced; the expression of the (R)-specific enoyl-CoA hydratase gene on the chromosome is enhanced; and the bktB structural gene (β-ketothiolase gene) and the A1528 structural gene (β-ketothiolase gene) are deleted.

### (Example 1)

### PHA production by PHA copolymer mixture-producing microbial strain (1)

Culture examination using the PHA copolymer mixture-producing microbial strain (1) was conducted under the conditions described below.

### (Culture Media)

The seed culture medium was composed of 1 w/v% Meat-extract, 1 w/v% Bacto-Tryptone, 0.2 w/v% Yeast-extract, 0.9 w/v% Na₂HPO₄•12H₂O, and 0.15 w/v% KH₂PO₄ (pH = 6.8).

The preculture medium was composed of 1.1 w/v% Na₂HPO₄•12H₂O, 0.19 w/v% KH₂PO₄, 1.29 w/v% (NH₄)₂SO₄, 0.1 w/v% MgSO₄•7H₂O, 2.5 w/v% palm olein oil, and 0.5 v/v% trace metal salt solution (solution of 1.6 w/v% FeCl₃•6H₂O, 1 w/v% CaCl₂•2H₂O, 0.02 w/v% CoCl₂•6H₂O, 0.016 w/v% CuSO₄•5H₂O, and 0.012 w/v% NiCl₂•6H₂O in 0.1N hydrochloric acid).

The PHA production culture medium was composed of 0.385 w/v% Na₂HPO₄•12H₂O, 0.067 w/v% KH₂PO₄, 0.291 w/v% (NH₄)₂SO₄, 0.1 w/v% MgSO₄•7H₂O, and 0.5 v/v% trace metal salt solution (solution of 1.6 w/v% FeCl₃•6H₂O, 1 w/v% CaCl₂•2H₂O, 0.02 w/v% CoCl₂•6H₂O, 0.016 w/v% CuSO₄•5H₂O, and 0.012 w/v% NiCl₂•6H₂O in 0.1N hydrochloric acid).

### (Method of Measuring Percentage of Accumulated PHA)

The percentage of accumulated PHA to dried microbial bodies was measured as follows. The microbial bodies were collected from the culture fluid by centrifugation. The collected microbial bodies were washed with ethanol and freeze-dried to give dried microbial bodies, the weight of which was measured. To 1 g of the dried microbial bodies was added 100 ml of chloroform, and the microbial bodies in chloroform were stirred at room temperature for a day to extract a PHA (PHA copolymer mixture) from the microbial bodies. The residual microbial bodies were removed by filtration, and the filtrate was concentrated using an evaporator to a total volume of 30 ml. To the concentrate was slowly added 90 ml of hexane, and the liquid mixture was left for 1 hour under gentle stirring. The PHA precipitated was collected by filtration and vacuum-dried at 50°C for 3 hours. The weight of the dried PHA was measured, and the percentage of the accumulated PHA to the dried microbial bodies was calculated.

### (Method of Measuring Weight Percentages of PHA fractions (I) and (II))

The weight percentages of the PHA fractions (1) and (II) in the PHA copolymer mixture were measured as follows. First, the dried PHA was fractionated into the PHA fractions (I) and (II) by the MIBK fractionation described above, and the weight of each of the fractions (I) and (II) was measured. Subsequently, the percentage of the weight of each of the PHA fractions (I) and (II) to the total weight of the PHA fractions (I) and (II) was calculated.

### (Method of Measuring Average 3HH Unit Ratio)

The average 3HH unit ratio of each of the PHA copolymer mixture, PHA fraction (I), and PHA fraction (II) was measured as follows. To about 20 mg of the dried PHA copolymer mixture, PHA fraction (1), or PHA fraction (II) were added 1 ml of a sulfuric acid-methanol mixture (15:85) and 1 ml of chloroform, and the receptacle was hermetically closed. The contents of the receptacle were heated at 100°C for 140 minutes to give a methyl ester of a PHA degradant. After cooling, 0.5 ml of deionized water was added, and the contents of the receptacle were thoroughly mixed and then left until separation into aqueous and organic layers was completed. Subsequently, the organic layer was separately collected, and the monomer unit composition of the PHA degradant in the collected organic layer was analyzed by capillary gas chromatography. The gas chromatograph used was GC-17 A manufactured by Shimadzu Corporation, and the capillary column used was NEUTRA BOND-1 manufactured by GL Sciences Inc. (column length = 25 m, column inner diameter = 0.25 mm, liquid film thickness = 0.4 µm). The carrier gas used was He, the column inlet pressure was 100 kPa, and the amount of the injected sample was 1 µl. As for the temperature conditions, the temperature was 50°C at the beginning, increased to 200°C at a rate of 8°C/min, and further increased from 200 to 290°C at a rate of 30°C/min. The average 3HH unit ratio of the PHA copolymer mixture, PHA fraction (I), or PHA fraction (II) was calculated based on peaks observed in the analysis under the above conditions.

### (Method of Measuring Melting Peak Temperature of PHA Copolymer Mixture)

About 2 mg of the PHA copolymer mixture was weighed and subjected to differential scanning calorimetry which used a differential scanning calorimeter (DSC 8500 manufactured by PerkinElmer Inc.) and in which the PHA copolymer mixture was heated from -30 to 200°C at a rate of 10°C/min. In the DSC curve obtained by the calorimetry, the temperature was determined at which a melting peak exhibiting a melting enthalpy of 0.5 J/g or more was detected.

### (Evaluation of Processability of PHA Copolymer Mixture and Production of Pellets)

An amount of 4.5 g of the PHA copolymer mixture was placed into a small-sized kneader (DSM Xplore 5, model 2005, manufactured by DSM), into which 0.045 g of pentaerythritol (Neulizer P manufactured by Mitsubishi Chemical Corporation), 0.0225 g of behenamide (BNT-22 H manufactured by Nippon Fine Chemical Co., Ltd.), and 0.0225 g of erucamide (NEUTRON-S manufactured by Nippon Fine Chemical Co., Ltd.) were added as additives. The PHA copolymer mixture and the additives were kneaded at a barrel temperature of 170°C and a screw rotational speed of 100 rpm for 5 minutes. After the kneading, a molten resin composition in the shape of a strand was discharged through a die and immediately placed into a water bath heated to 60°C, and the time taken for crystallization and solidification of the strand was measured. In the case where the strand was solidified within 100 seconds, the processability was rated good.

After that, the strand crystallized and solidified in the water bath was cut with nipper into resin composition pellets.

### (Evaluation of Tearing Resistance)

A PET film (thickness = 50 µm) having one surface subjected to mold release treatment was placed on a 2-mm-thick SUS plate (30 cm × 35 cm) in a position where the treated surface faced away from the SUS plate, and 1.3 g of the resin composition pellets were placed on the PET film. A 70-µm-thick shim plate was placed as a spacer to surround the resin composition pellets. After that, a plate identical to the above SUS plate was placed to sandwich the resin composition pellets. The sandwiched pellets were placed on the hot press plate of a press machine (Compression Molding Machine NSF-50, manufactured by Sinto Metal Industries, Ltd.) which was heated to 160°C, and were preheated for 5 minutes. After the preheating, the pressure was gradually increased to 5 MPa over 2 minutes, and the increased pressure was maintained for 2 minutes. The pressing was followed by cooling to a room temperature on a cooling plate cooled to about 20°C, and thus an about 50-µm-thick film was obtained. This film was aged at a room temperature of 23°C and a humidity of 50% for 1 week, and the resulting film was used as a film sample.

The film sample was tested using a light load-type tearing tester (NO. 2037 special model, manufactured by Kumagai Riki Kogyo Co., Ltd.) having a function and structure conforming with those of a standard Elmendorf tearing tester as specified in JIS P-8116, and the measurement value was divided by the thickness of the film sample. The resulting value was adopted as a measure of the Elmendorf tearing resistance of the film sample.

### (PHA Production Culture)

PHA production culture was performed as follows. First, a glycerol stock (50 µl) of the PHA copolymer mixture-producing microbial strain (1) was inoculated into the seed culture medium (10 ml) and cultured for 24 hours to accomplish seed culture. Subsequently, the seed culture fluid was inoculated at a concentration of 1.0 v/v% into a 3L jar fermenter (MDL-300, manufactured by B.E. Marubishi Co., Ltd.) containing 1.8 L of the preculture medium. The fermenter was operated at a culture temperature of 30°C, a stirring speed of 500 rpm, and an aeration of 1.8 L/min, and the preculture was conducted for 28 hours during which the pH was controlled between 6.7 and 6.8. For the pH control, a 14% aqueous solution of ammonium hydroxide was used.

Next, the preculture fluid was inoculated at a concentration of 5.0 v/v% into a 5Ljar fermenter (MDS-U50, manufactured by B.E. Marubishi Co., Ltd.) containing 2.5 L of the PHA production culture medium. The fermenter was operated at a culture temperature of 33°C, a stirring speed of 420 rpm, and an aeration of 2.1 L/min, and the pH was controlled between 6.7 and 6.8. For the pH control, a 25% aqueous solution of ammonium hydroxide was used. The carbon source was added intermittently. Palm olein oil was used as the carbon source. The culture was continued until the percentage of accumulated PHA to dried microbial bodies reached 80% or more. The percentage of accumulated PHA to dried microbial bodies, the average 3HH unit ratios of the PHA copolymer mixture, PHA fraction (I), and PHA fraction (II), the weight percentages of the PHA fractions (I) and (II), the melting peak temperature, the melting enthalpy, the processability, and the Elmendorf tearing resistance were measured as described above. The results are shown in Table 1.

### (Example 2)

### PHA production by PHA copolymer mixture-producing microbial strain (2)

Culture examination using the PHA copolymer mixture-producing microbial strain (2) was conducted under the same conditions as the culture examination in Example 1. The percentage of accumulated PHA to dried microbial bodies, the average 3HH unit ratios of the PHA copolymer mixture, PHA fraction (I), and PHA fraction (II), the weight percentages of the PHA fractions (I) and (II), the melting peak temperature, the melting enthalpy, the processability, and the Elmendorf tearing resistance are shown in Table 1.

### (Example 3)

### PHA production by PHA copolymer mixture-producing microbial strain (3)

Culture examination using the PHA copolymer mixture-producing microbial strain (3) was conducted under the same conditions as the culture examination in Example 1. The percentage of accumulated PHA to dried microbial bodies, the average 3HH unit ratios of the PHA copolymer mixture, PHA fraction (I), and PHA fraction (II), the weight percentages of the PHA fractions (I) and (II), the melting peak temperature, the melting enthalpy, the processability, and the Elmendorf tearing resistance are shown in Table 1.

### (Example 4)

### PHA production by PHA copolymer mixture-producing microbial strain (4)

Culture examination using the PHA copolymer mixture-producing microbial strain (4) was conducted under the same conditions as the culture examination in Example 1. The percentage of accumulated PHA to dried microbial bodies, the average 3HH unit ratios of the PHA copolymer mixture, PHA fraction (I), and PHA fraction (II), the weight percentages of the PHA fractions (I) and (II), the melting peak temperature, the melting enthalpy, the processability, and the Elmendorf tearing resistance are shown in Table 1.

### (Example 5)

### PHA production by PHA copolymer mixture-producing microbial strain (5)

Culture examination using the PHA copolymer mixture-producing microbial strain (5) was conducted under the same conditions as the culture examination in Example 1. The percentage of accumulated PHA to dried microbial bodies, the average 3HH unit ratios of the PHA copolymer mixture, PHA fraction (I), and PHA fraction (II), the weight percentages of the PHA fractions (I) and (II), the melting peak temperature, the melting enthalpy, the processability, and the Elmendorf tearing resistance are shown in Table 1.

### (Example 6)

### PHA production by PHA copolymer mixture-producing microbial strain (6)

Culture examination using the PHA copolymer mixture-producing microbial strain (6) was conducted under the same conditions as the culture examination in Example 1. The percentage of accumulated PHA to dried microbial bodies, the average 3HH unit ratios of the PHA copolymer mixture, PHA fraction (I), and PHA fraction (II), the weight percentages of the PHA fractions (I) and (II), the melting peak temperature, the melting enthalpy, the processability, and the Elmendorf tearing resistance are shown in Table 1.

### (Comparative Example 1)

### PHA production by P(3HB-co-3HH)-producing microbial strain (1)

Culture examination using the P(3HB-co-3HH)-producing microbial strain (1) was conducted under the same conditions as the culture examination in Example 1. The percentage of accumulated PHA to dried microbial bodies, the average 3HH unit ratios of P(3HB-co-3HH), MIBK-soluble fraction, and MIBK-insoluble fraction, the weight percentages of the MIBK-soluble and MIBK-insoluble fractions, the melting peak temperature, the melting enthalpy, the processability, and the Elmendorf tearing resistance are shown in Table 1. In the analysis, a PHA accumulated by the P(3HB-co-3HH)-producing microbial strain (1) was used instead of the PHA copolymer mixture. The PHA accumulated by the P(3HB-co-3HH)-producing microbial strain (1) was P(3HB-co-3HH). The MIBK-soluble fraction and the MIBK-insoluble fraction were fractions obtained in the same manner as the PHA fraction (I) and the PHA fraction (II), respectively.

### (Comparative Example 2)

### PHA production by P(3HB-co-3HH)-producing microbial strain (2)

Culture examination using the P(3HB-co-3HH)-producing microbial strain (2) was conducted under the same conditions as the culture examination in Example 1. The percentage of accumulated PHA to dried microbial bodies, the average 3HH unit ratios of P(3HB-co-3HH), MIBK-soluble fraction, and MIBK-insoluble fraction, the weight percentages of the MIBK-soluble and MIBK-insoluble fractions, the melting peak temperature, the melting enthalpy, the processability, and the Elmendorf tearing resistance are shown in Table 1. In the analysis, a PHA accumulated by the P(3HB-co-3HH)-producing microbial strain (2) was used instead of the PHA copolymer mixture. The PHA accumulated by the P(3HB-co-3HH)-producing microbial strain (2) was P(3HB-co-3HH). The MIBK-soluble fraction and the MIBK-insoluble fraction were fractions obtained in the same manner as the PHA fraction (I) and the PHA fraction (II), respectively.

### (Comparative Example 3)

### PHA production by P(3HB-co-3HH)-producing microbial strain (3)

Culture examination using the P(3HB-co-3HH)-producing microbial strain (3) was conducted under the same conditions as the culture examination in Example 1. The percentage of accumulated PHA to dried microbial bodies, the average 3HH unit ratios of P(3HB-co-3HH), MIBK-soluble fraction, and MIBK-insoluble fraction, the weight percentages of the MIBK-soluble and MIBK-insoluble fractions, the melting peak temperature, the melting enthalpy, the processability, and the Elmendorf tearing resistance are shown in Table 1. In the analysis, a PHA accumulated by the P(3HB-co-3HH)-producing microbial strain (3) was used instead of the PHA copolymer mixture. The PHA accumulated by the P(3HB-co-3HH)-producing microbial strain (3) was P(3HB-co-3HH). The MIBK-soluble fraction and the MIBK-insoluble fraction were fractions obtained in the same manner as the PHA fraction (I) and the PHA fraction (II), respectively.

### (Comparative Example 4)

### PHA production by P(3HB-co-3HH)-producing microbial strain (4)

Culture examination using the P(3HB-co-3HH)-producing microbial strain (4) was conducted under the same conditions as the culture examination in Example 1. The percentage of accumulated PHA to dried microbial bodies, the average 3HH unit ratios of P(3HB-co-3HH), MIBK-soluble fraction, and MIBK-insoluble fraction, the weight percentages of the MIBK-soluble and MIBK-insoluble fractions, the melting peak temperature, the melting enthalpy, the processability, and the Elmendorf tearing resistance are shown in Table 1. In the analysis, a PHA accumulated by the P(3HB-co-3HH)-producing microbial strain (4) was used instead of the PHA copolymer mixture. The PHA accumulated by the P(3HB-co-3HH)-producing microbial strain (4) was P(3HB-co-3HH). The MIBK-soluble fraction and the MIBK-insoluble fraction were fractions obtained in the same manner as the PHA fraction (I) and the PHA fraction (II), respectively.

**[Table 1]**

| | PHA copolymer mixture-producing microbial strain | Percentage of accumulated PHA (%) | PHA copolymer mixture | | PHA fraction (I) | | PHA fraction (II) | | Processability | Elmendorf tearing resistance (N/mm) |
|---|---|---|---|---|---|---|---|---|---|---|
| | | | 3HH ratio (mol%) | Melting peak temperature (Melting enthalpy) | Weight percentage (%) | 3HH ratio (mol%) | Weight percentage (%) | 3HH ratio (mol%) | | |
| Example 1 | (1) | 88 | 13.3 | 44.7°C (1.3 J/g) | 31 | 28.1 | 69 | 6.4 | Good | 15.5 |
| | | | | 73.2°C (0.5 J/g) | | | | | | |
| | | | | 152.3°C (48.3 J/g) | | | | | | |
| Example 2 | (2) | 90 | 15.7 | 45.5°C (2.2 J/g) | 44 | 29.1 | 56 | 4.9 | Good | 19.7 |
| | | | | 67.0°C (0.7 J/g) | | | | | | |
| | | | | 152.3°C (36.6 J/g) | | | | | | |
| Example 3 | (3) | 91 | 17.8 | 44.7°C (4.4 J/g) | 61 | 24.1 | 39 | 7.6 | Good | 69.7 |
| | | | | 72.2°C (3.1 J/g) | | | | | | |
| | | | | 154.1°C (12.3 J/g) | | | | | | |
| Example 4 | (4) | 90 | 19.9 | 45.4°C (7.1 J/g) | 68 | 25.2 | 32 | 9.9 | Good | 72.0 |
| | | | | 65.3°C (2.1 J/g) | | | | | | |
| | | | | 152.3°C (6.4 J/g) | | | | | | |
| Example 5 | (5) | 88 | 16.2 | 63.4°C (2.1 J/g) | 73 | 20.2 | 27 | 5.4 | Good | 23.8 |
| | | | | 80.0°C (6.5 J/g) | | | | | | |
| | | | | 165.0°C (14.6 J/g) | | | | | | |
| Example 6 | (6) | 86 | 15.3 | 43.8°C (1.3 J/g) | 44 | 30.7 | 56 | 3.7 | Good | 30.1 |
| | | | | 67.9°C (1.1 J/g) | | | | | | |
| | | | | 164.5°C (38.3 J/g) | | | | | | |

| | P(3HB-co-3HH)-producing microbial strain | Percentage of accumulated PHA (%) | P(3HB-co-3HH) | | MIBK-soluble fraction | | MIBK-insoluble fraction | | Processability | Elmendorf tearing resistance (N/mm) |
|---|---|---|---|---|---|---|---|---|---|---|
| | | | 3HH ratio (mol%) | Melting peak temperature (Melting enthalpy) | Weight percentage (%) | 3HH ratio (mol%) | Weight percentage (%) | 3HH ratio (mol%) | | |
| Comparative Example 1 | (1) | 90 | 5.7 | 130.3°C (6.0 J/g) | 0 | - | 100 | 5.7 | Good | 1.3 |
| | | | | 143.3°C (14.7 J/g) | | | | | | |
| Comparative Example 2 | (2) | 89 | 17.2 | 48.9°C (4.9 J/g) | 84 | 18.4 | 16 | 11.2 | Poor | 5.3 |
| | | | | 87.9°C (5.9 J/g) | | | | | | |
| Comparative Example 3 | (3) | 84 | 24.6 | 44.7°C (8.1 J/g) | 100 | 24.6 | 0 | - | Poor | 45.5 |
| Comparative Example 4 | (4) | 76 | 28.2 | 45.5°C (11.9 J/g) | 100 | 28.2 | 0 | - | Poor | 60.5 |

Table 1 reveals that the PHA copolymer mixtures obtained in Examples 1 to 6 had good processability, high Elmendorf tearing resistance, and excellent mechanical properties. In Comparative Example 1, where the PHA copolymer mixture obtained did not contain the PHA fraction (I), the Elmendorf tearing resistance was low. In Comparative Example 2, where the average 3HH unit ratio of the PHA fraction (I) was less than 20 mol%, the processability was poor, and the Elmendorf tearing resistance was low. In Comparative Examples 3 and 4, where the PHA copolymer mixture had an average 3HH unit ratio of more than 22 mol% and did not contain the PHA fraction (II), the processability was poor.

## Claims

1. A method of producing a polyhydroxyalkanoate copolymer mixture, the method comprising the step of culturing a microorganism that produces the polyhydroxyalkanoate copolymer mixture, wherein
the polyhydroxyalkanoate copolymer mixture comprises:
a polyhydroxyalkanoate fraction (I) comprising a polyhydroxyalkanoate copolymer having a 3-hydroxybutyrate structural unit and a 3-hydroxyhexanoate structural unit, the polyhydroxyalkanoate fraction (I) having an average 3-hydroxyhexanoate unit ratio of 20 mol% or more; and
a polyhydroxyalkanoate fraction (II) comprising a polyhydroxyalkanoate having a 3-hydroxybutyrate structural unit, the polyhydroxyalkanoate fraction (II) having an average 3-hydroxyhexanoate unit ratio of 0 to 15 mol%, and
the polyhydroxyalkanoate copolymer mixture has an average 3-hydroxyhexanoate unit ratio of 22 mol% or less.

2. The method according to claim 1, wherein a weight percentage of the polyhydroxyalkanoate fraction (I) in the polyhydroxyalkanoate copolymer mixture is from 10 to 90%.

3. The method according to claim 1 or 2, wherein the average 3-hydroxyhexanoate unit ratio of the polyhydroxyalkanoate copolymer mixture is from 10 to 22 mol%.

4. The method according to any one of claims 1 to 3, wherein the microorganism has genes encoding two types of polyhydroxyalkanoate synthases differing in polymerization activity for 3-hydroxyhexanoyl-CoA.

5. The method according to claim 4, wherein amino acid sequences of the two types of polyhydroxyalkanoate synthases differing in polymerization activity for 3-hydroxyhexanoyl-CoA have a sequence identity of 90% or less.

6. The method according to claim 4 or 5, wherein the genes encoding the two types of polyhydroxyalkanoate synthases differing in polymerization activity for 3-hydroxyhexanoyl-CoA include:
a gene (A) encoding a polyhydroxyalkanoate synthase having higher polymerization activity for 3-hydroxyhexanoyl-CoA than an *Aeromonas caviae-*derived wild-type polyhydroxyalkanoate synthase having an amino acid sequence of SEQ ID NO: 1; and
a gene (B) encoding a polyhydroxyalkanoate synthase having lower polymerization activity for 3-hydroxyhexanoyl-CoA than the *Aeromonas caviae-*derived wild-type polyhydroxyalkanoate synthase.

7. The method according to claim 6, wherein the gene (A) is a polyhydroxyalkanoate synthase gene derived from a microorganism of the genus *Aeromonas* or a mutant of the polyhydroxyalkanoate synthase gene.

8. The method according to claim 7, wherein the gene (A) is a gene encoding an amino acid sequence having a sequence identity of 99.5 to 100% with an amino acid sequence of SEQ ID NO: 2 or 3.

9. The method according to any one of claims 6 to 8, wherein the gene (B) is composed of a combination of a part of a polyhydroxyalkanoate synthase gene derived from a microorganism of the genus *Aeromonas* and a part of a polyhydroxyalkanoate synthase gene derived from a microorganism of the genus *Cupriavidus.*

10. The method according to claim 9, wherein the gene (B) is a gene encoding an amino acid sequence having a sequence identity of 90 to 100% with an amino acid sequence of SEQ ID NO: 6.

11. The method according to any one of claims 6 to 8, wherein the gene (B) is a polyhydroxyalkanoate synthase gene derived from a microorganism of the genus *Chromobacterium* or a mutant of the polyhydroxyalkanoate synthase gene.

12. The method according to claim 11, wherein the gene (B) is a gene encoding an amino acid sequence having a sequence identity of 90 to 100% with an amino acid sequence of SEQ ID NO: 4 or 5.

13. The method according to any one of claims 6 to 8, wherein the gene (B) is a polyhydroxyalkanoate synthase gene derived from a microorganism of the genus *Bacillus* or a mutant of the polyhydroxyalkanoate synthase gene.

14. The method according to claim 13, wherein the gene (B) is a gene encoding amino acid sequences having a sequence identity of 90 to 100% with amino acid sequences of SEQ ID NOS: 7 and 8.

15. The method according to any one of claims 1 to 14, wherein the microorganism is a transformed microorganism that has been transformed to supply a greater amount of 3-hydroxyhexanoyl-CoA to an intracellular polyhydroxyalkanoate synthase than a wild strain of the microorganism.

16. The method according to claim 15, wherein the transformed microorganism has been transformed to inhibit degradation of an intermediate metabolite having six carbon atoms in β-oxidation of an oil or a fatty acid.

17. The method according to claim 16, wherein the transformed microorganism has been transformed to inhibit expression of a gene encoding a β-ketothiolase enzyme having thiolysis activity for β-ketohexanoyl-CoA which is β-ketoacyl-CoA having six carbon atoms.

18. The method according to claim 17, wherein the β-ketothiolase enzyme has an amino acid sequence having a sequence identity of 90 to 100% with an amino acid sequence of SEQ ID NO: 9 or 10.

19. The method according to any one of claims 1 to 18, wherein the microorganism has a gene encoding a protein having (R)-specific enoyl-CoA hydratase activity.

20. The method according to any one of claims 1 to 19, wherein in the culturing step, a carbon source containing an oil or a fatty acid is added.

21. The method according to claim 20, wherein the carbon source containing an oil or a fatty acid is a carbon source containing a middle-chain fatty acid having 6 to 12 carbon atoms or a glyceride of the middle-chain fatty acid.

22. The method according to claim 21, wherein the middle-chain fatty acid is hexanoic acid.

23. The method according to any one of claims 1 to 22, wherein the microorganism belongs to the genus *Cupriavidus* or is a result of transformation of a microorganism of the genus *Cupriavidus.*

24. The method according to claim 23, wherein the microorganism is *Cupriavidus necator* or transformed *Cupriavidus necator.*

25. A transformed microorganism that produces a polyhydroxyalkanoate copolymer mixture, the transformed microorganism comprising a gene encoding two types of polyhydroxyalkanoate synthases differing in polymerization activity for 3-hydroxyhexanoyl-CoA, wherein
the transformed microorganism has been transformed to supply a greater amount of 3-hydroxyhexanoyl-CoA to an intracellular polyhydroxyalkanoate synthase than a wild strain of the transformed microorganism,
the polyhydroxyalkanoate copolymer mixture comprises:
a polyhydroxyalkanoate fraction (I) comprising a polyhydroxyalkanoate copolymer having a 3-hydroxybutyrate structural unit and a 3-hydroxyhexanoate structural unit, the polyhydroxyalkanoate fraction (I) having an average 3-hydroxyhexanoate unit ratio of 20 mol% or more; and
a polyhydroxyalkanoate fraction (II) comprising a polyhydroxyalkanoate having a 3-hydroxybutyrate structural unit, the polyhydroxyalkanoate fraction (II) having an average 3-hydroxyhexanoate unit ratio of 0 to 15 mol%, and
the polyhydroxyalkanoate copolymer mixture has an average 3-hydroxyhexanoate unit ratio of 22 mol% or less.
